# EUROPEAN PATENT SPECIFICATION

(11) **EP 3 771 454 B1**
(45) Date of publication and mention of the grant of the patent: **16.11.2022**
(21) Application number: 19220018.6
(22) Date of filing: 30.12.2019
(51) Int. Cl.: A61F 2/18, A61C 8/02, A61L 31/00, A61L 31/04

(54) **SEALING DEVICE FOR REPAIRING PERFORATION OF MAXILLARY SINUS MUCOSA**
DICHTUNGSVORRICHTUNG ZUR REPARATUR DER PERFORATION DER SCHLEIMHAUT VON SINUS MAXILLARIS
DISPOSITIF D'ÉTANCHÉITÉ POUR LA RÉPARATION D'UNE PERFORATION DE LA MUQUEUSE DU SINUS MAXILLAIRE

(30) Priority: 29.07.2019 CN 201921206721 U
(43) Date of publication of application: 03.02.2021
(73) Proprietor: Shanghai Ninth Peoples Hospital Affiliated Shanghai Jiaotong University School of Medicine, Shanghai City (CN)
(72) Inventor: ZOU, Duohong, Shanghai City (CN); XU, Guangzhou, Shanghai City (CN); HU, Yingkai, Shanghai City (CN); YANG, Chi, Shanghai City (CN)
(74) Representative: Zimmermann, Tankred Klaus

(56) References cited:
- DE-C1- 4 321 785
- US-A- 5 547 378
- US-A1- 2005 102 036
- US-A1- 2006 287 732

## Description

### BACKGROUND OF THE INVENTION

### 1. Field of the Invention

The present invention relates to a medical apparatus and instruments technology field, and more particularly to a sealing device for repairing perforation of maxillary sinus mucosa.

### 2. Description of the Prior Art

With the development of dental implant technology, it has become a routine treatment to reconstruct the oral function with dental implants. The maxillary sinus is adjacent to the maxillary molar region, due to anatomical factors, physiological absorption of alveolar bone, inflammation, and maxillary sinus gasification, it often leads to insufficient vertical bone mass in the maxillary molar region, which poses a challenge to the maxillary molar implant. Maxillary sinus lift is the most effective treatment for severe atrophy of the alveolar ridge and vertical bone loss in the maxillary posterior region. It is often used in cases of maxillary posterior alveolar bone height less than 5mm. In the process of maxillary sinus lift, maxillary sinus mucosal perforation is a serious and common complication. The average incidence in the literature is 19.5% (3.6%~58.3%). The main causes include inflammation of the maxillary sinus, smoking maxillary sinus mucosal thinning, bone separation at the sinus floor, insufficient residual bone height, cysts, and improper operation. The key to the perforation of the maxillary sinus mucosa is to seal the perforated maxillary sinus so that the bone powder can be stably maintained in the bone graft area without leaking into the maxillary sinus cavity, otherwise the maxillary sinus inflammation and bone graft failure will be caused. When the perforation is small, the maxillary sinus mucosa can be naturally contacted after being lifted up, and the perforation is covered with a Bio-Gide collagen membrane, and then the bone powder can be implanted. When the perforation is large, the existing biofilm cannot guarantee tight coverage of the perforation, so the surgery will be abandoned, and after at least 2 to 3 months, treatment will be applied. This not only lengthens the treatment cycle, increases the number of operations, but also increases the patient's pain and financial burden. In the second operation, the mucosa may be more difficult to peel off due to scar formation so as to cause perforation again.

In 2015, the population of China over 60 years old has exceeded 222 million people. It is estimated that by 2020, the elder population will exceed 250 million people. The maxillary sinus gasification (up to 83.2%) and alveolar bone resorption due to tooth loss make many elder people with insufficient bone mass in the maxillary posterior region; it is required to perform maxillary sinus lift. In addition, the incidence of perforation of the maxillary sinus mucosa is about 20%. Therefore, it needs to provide a new sealing device that can tightly seal the perforation of any diameter of the maxillary sinus mucosa to prevent leakage of bone powder into the maxillary sinus cavity.

Bio-Gide^{®} (Geistlich, Switzerland) collagen membrane is currently the most widely used absorbable bio-collagen membrane. It is formed by processing porcine dermal type I collagen and type III collagen. It has good bio-compatibility and has a two-layer structure. The fiber of one layer structure is dense and has good barrier effect; the other layer structure has more pores, which is beneficial to the adhesion of osteoblast to the membrane. However, they are all flat membrane structures, with 13 × 25mm, 25 × 25mm and 30 × 40mm three models, which are difficult to operate when repairing large perforations of the maxillary sinus mucosa.

Document US5547378 shows a collagen balloon filled with bone chips for closing a pierced sinus membrane.

### BRIEF SUMMARY OF THE INVENTION

One object of the present invention is to provide a sealing device for repairing perforation of the maxillary sinus mucosa, which can tightly seal the perforation of any diameter of the maxillary sinus mucosa, and can prevent the bone powder from leaking into the maxillary sinus cavity, and can complete the maxillary sinus lift one-off.

In order to achieve the above object, the present invention provides a sealing device for repairing perforation of maxillary sinus mucosa, the sealing device is a bag-like structure, comprises a bag mouth and a bag body, and consist of an absorbable bio-collagen membrane having a rough surface on one side and a smooth surface on the other side, wherein the bag mouth is a stitching portion, an inner wall of the bag body forms the rough surface, and an outer wall of the bag body forms the smooth surface.

As a preferred solution, the bag mouth is thickened.

As a preferred solution, the absorbable bio-collagen membrane is Bio-Gide membrane.

As a preferred solution, the volume of the sealing device is 3 cm³ - 5 cm³.

As a preferred solution, the bag body is filled with bone powder.

As a preferred solution, the bag mouth is provided with a tightening device.

The present invention has the advantage that the sealing device provided by the present invention for repairing the maxillary sinus mucosal perforation can tightly seal the perforation of any diameter of the maxillary sinus mucosa, prevent bone powder from leaking into the maxillary sinus cavity, and exploit the stability of the implanted material to realize completeness with the maxillary sinus lift operation in one time, without waiting for a second operation after the mucosa has healed, thereby shortening the treatment course, and reducing the patient's pain and economic burden.

### BRIEF DESCRIPTION OF THE DRAWINGS

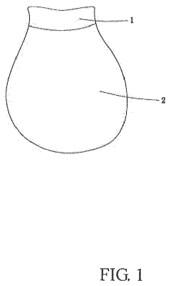

FIG. 1 is a structure schematic view of a sealing device for repairing perforation of maxillary sinus mucosa.

### DETAILED DESCRIPTION OF THE PREFERRED EMBODIMENTS

The following text will describe embodiments of the present invention in detailed. The embodiments are shown in the accompanying drawings, in which the same or similar signs represent the same or similar elements or elements with the same or similar functions from beginning to end. The following embodiments described with reference to the accompanying drawings are illustrative and are intended only to explain the present invention and are not understood as limitations to the invention.

Please refer to FIG. 1, a sealing device for repairing perforation of maxillary sinus mucosa is a bag-like structure, comprises a bag mouth 1 and a bag body 2 and consists of an absorbable bio-collagen membrane having a rough surface on one side and a smooth surface on the other side, wherein the bag mouth is a stitching portion; an inner wall of the bag body forms the rough surface and an outer wall of the bag body forms the smooth surface.

In order to facilitate the suture to fix it to the oral mucosa, the bag mouth 1 is thickened.

The absorbable bio-collagen membrane is preferred as Bio-Gide membrane.

Bio-Gide^{®} (Geistlich, Switzerland) collagen membrane is currently the most widely used absorbable bio-collagen membrane. It is formed by processing porcine dermal type I collagen and type III collagen. It has good bio-compatibility and has a two-layer structure. The fiber of one layer structure is dense and has good barrier effect; the other layer structure has more pores, which is beneficial to the adhesion of osteoblast and membrane.

As a preferred solution, the volume of the sealing device is 3 cm³ - 5 cm³. The volume of the maxillary sinus varies widely and an average value is about 15 cm³. The volume of the maxillary sinus of male is slightly larger than that of female (18 vs 14.1 cm³) (Sahlstrand-Johnson P, Jannert M, Strömbeck A, et al. BMC Med Imaging , 2011, 11: 8.). When the maxillary sinus is raised outside, the bone graft area is generally within 1/3 of the space below the maxillary sinus. Therefore, three types of bag biofilms are designed with a volume of 3 cm³, 4 cm³, and 5 cm³, and the size of the biofilm is 15 × 20 mm, 20 × 25 mm, and 25 × 30 mm when tiled used in patients with different levels of bone defects.

As a preferred solution, bone powder is put in the bag body 2.

As a preferred solution, the bag mouth 1 is provided with a tightening device. The tightening device is used to directly tighten the bag mouth 1, which can be a pull line.

The maxillary sinus mucosal perforation is occurred when the maxillary sinus is lifted externally, the sealing device for repairing the maxillary sinus mucosal perforation of the present invention is firstly put into so that the outer wall of the sealing device contacts with the maxillary sinus mucosa and bone wall therefor sealing the maxillary sinus mucosal perforation. Bone graft material is filled in from the bag mouth. The bag mouth 1 is fixed to the oral mucosa near the bone surface by sutures. The surface of the bone powder at the window of the maxillary sinus is covered with Bio-Gide collagen membrane to isolate the bone powder and oral mucosa.

The above description of the present invention is described in detail. In above text, the principles and implementation mode of the present invention are expounded with specific examples, and above specific examples are only for helping to understand the invention. The scope of the present invention is defined by the claims.

## Claims

1. A sealing device for repairing perforation of maxillary sinus mucosa, wherein:
the sealing device is a bag-like structure, comprises a bag mouth (1) and a bag body (2), and consist of an absorbable bio-collagen membrane having a rough surface on one side and a smooth surface on the other side, wherein the bag mouth (1) is a stitching portion, an inner wall of the bag body (2) forms the rough surface, and an outer wall of the bag body (2) forms the smooth surface.

2. The sealing device for repairing perforation of maxillary sinus mucosa as claimed in Claim 1, **characterized in that**: the bag mouth (1) is thickened.

3. The sealing device for repairing perforation of maxillary sinus mucosa as claimed in Claim 1, **characterized in that**: the volume of the sealing device is 3 cm³ - 5 cm³.

4. The sealing device for repairing perforation of maxillary sinus mucosa as claimed in Claim 1, **characterized in that**: the bag body (2) is filled with bone powder.

5. The sealing device for repairing perforation of maxillary sinus mucosa as claimed in Claim 1, **characterized in that**: the bag mouth (1) is provided with a tightening device.

## Patentansprüche

1. Abdichtungsvorrichtung zum Reparieren einer Perforation der Kiefernhöhlenschleimhaut, wobei:
die Abdichtungsvorrichtung eine beutelartige Struktur ist, eine Beutelöffnung (1) und einen Beutelkörper (2) aufweist, und aus einer absorbierbaren Biokollagenmembran besteht, die eine raue Oberfläche auf einer Seite und eine glatte Oberfläche auf der anderen Seite aufweist, wobei die Beutelöffnung (1) ein Nahtabschnitt ist, eine innere Wand des Beutelkörpers (2) die raue Oberfläche bildet, und eine äußere Wand des Beutelkörpers (2) die glatte Oberfläche bildet.

2. Die Abdichtungsvorrichtung zum Reparieren einer Perforation der Kiefernhöhlenschleimhaut gemäß Anspruch 1, die **dadurch gekennzeichnet ist, dass**: die Beutelöffnung (1) verdickt ist.

3. Die Abdichtungsvorrichtung zum Reparieren einer Perforation der Kiefernhöhlenschleimhaut gemäß Anspruch 1, die **dadurch gekennzeichnet ist, dass**: das Volumen der Abdichtungsvorrichtung 3 cm³ bis 5 cm³ ist.

4. Die Abdichtungsvorrichtung zum Reparieren einer Perforation der Kiefernhöhlenschleimhaut gemäß Anspruch 1, die **dadurch gekennzeichnet ist, dass**: der Beutelkörper (2) mit Knochenmehl gefüllt ist.

5. Die Abdichtungsvorrichtung zum Reparieren einer Perforation der Kiefernhöhlenschleimhaut gemäß Anspruch 1, die **dadurch gekennzeichnet ist, dass**: die Beutelöffnung (1) mit einer Spannvorrichtung versehen ist.

## Revendications

1. Dispositif d'étanchéité pour la réparation d'une perforation de la muqueuse du sinus maxillaire, dans lequel:
le dispositif d'étanchéité est une structure de type sachet, comprend une embouchure de sachet (1) et un corps de sachet (2), et se compose d'une membrane de bio-collagène résorbable présentant une surface rugueuse d'un côté et une surface lisse de l'autre côté, où l'embouchure de sachet (1) est une partie de couture, une paroi intérieure du corps de sachet (2) forme la surface rugueuse, et une paroi extérieure du corps de sachet (2) forme la surface lisse.

2. Dispositif d'étanchéité pour la réparation d'une perforation de la muqueuse du sinus maxillaire selon la revendication 1, **caractérisé par le fait que**: l'embouchure de sachet (1) est épaissie.

3. Dispositif d'étanchéité pour la réparation d'une perforation de la muqueuse du sinus maxillaire selon la revendication 1, **caractérisé par le fait que**: le volume du dispositif d'étanchéité est de 3 cm³ à 5 cm³.

4. Dispositif d'étanchéité pour la réparation d'une perforation de la muqueuse du sinus maxillaire selon la revendication 1, **caractérisé par le fait que**: le corps de sachet (2) est rempli de poudre d'os.

5. Dispositif d'étanchéité pour la réparation d'une perforation de la muqueuse du sinus maxillaire selon la revendication 1, **caractérisé par le fait que**: l'embouchure du sachet (1) est munie d'un dispositif de serrage.
